# EUROPÄISCHE PATENTSCHRIFT

(11) **EP 1 524 960 B1**
(45) Veröffentlichungstag und Bekanntmachung des Hinweises auf die Patenterteilung: **12.03.2008**
(21) Anmeldenummer: 03762551.4
(22) Anmeldetag: 30.06.2003
(51) Int. Cl.: A61Q 17/00, A61Q 19/00, A61K 8/49

(54) **KOSMETISCHE ODER DERMATOLOGISCHE ZUBEREITUNG ZUM SCHUTZ FOTOLABILER WIRKSTOFFE**
COSMETIC OR DERMATOLOGICAL PREPARATION FOR PROTECTING PHOTOLABILE ACTIVE INGREDIENTS
PREPARATION COSMETIQUE OU DERMATOLOGIQUE POUR PROTEGER DES SUBSTANCES ACTIVES PHOTOLABILES

(30) Priorität: 03.07.2002 DE 10229997
(43) Veröffentlichungstag der Anmeldung: 27.04.2005
(73) Patentinhaber: Beiersdorf AG, 20245 Hamburg (DE)
(72) Erfinder: BUERGER, Anette, 22303 Hamburg (DE); RASCHKE, Thomas, 25421 Pinneberg (DE)
(86) Internationale Anmeldenummer: PCT/EP2003/006910
(87) Internationale Veröffentlichungsnummer: WO 2004/004670

(56) Entgegenhaltungen:
- EP-A- 0 685 221
- WO-A-01/85123
- DE-A- 19 903 729

## Beschreibung

Die vorliegende Erfindung betrifft Stoffkombinationen zur Stabilisierung lichtempfindlicher Wirkstoffe sowie kosmetische und dermatologische Formulierungen mit auf diese Weise stabilisierten lichtempfindlichen Wirkstoffen. Insbesondere betrifft sie kosmetische und dermatologische Formulierungen mit den lichtempfindlichen Stoffen α-Liponsäure, Retinol und/oder oder mehrfach ungesättigte Fettsäuren, die durch den Einsatz dieser Stoffkombinationen stabilisiert werden.

Die vorliegende Erfindung betrifft dementsprechend in bevorzugten Ausführungsformen kosmetische bzw. dermatologische Zubereitungen, enthaltend Wirkstoffe zur Pflege und zum Schutz der Haut, insbesondere der empfindlichen Haut. Ganz besonders im Vordergrund steht die durch intrinsische und/oder extrinsische Faktoren gealterte oder alternde Haut sowie die Verwendung solcher Wirkstoffe und Kombinationen solcher Wirkstoffe auf dem Gebiete der kosmetischen und dermatologischen Hautpflege.

Kosmetische Hautpflege dient in erster Linie dazu, die natürliche Funktion der Haut als Barriere gegen Umwelteinflüsse, wie z. B. Schmutz, Chemikalien oder Mikroorganismen, und gegen den Verlust von körpereigenen Stoffen, wie Wasser, natürliche Fette oder Elektrolyte, zu stärken oder wiederherzustellen.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z. B. durch endogene, genetisch determinierte Faktoren verursacht. In Epidermis und Dermis kommt es alterungsbedingt zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z. B. nach Waschen).
   Exogene Faktoren, wie UV-Licht und chemische Noxen, können kumulativ wirksam sein und z. B. die endogenen Alterungsprozesse beschleunigen bzw. sie ergänzen. In Epidermis und Dermis kommt es insbesondere durch exogene Faktoren zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z. B. Altersflecken) und
g) vergrößerte Anfälligkeit gegenüber mechanischem Stress (z. B. Rissigkeit).

Es sind eine Vielzahl von Produkten und Wirkstoffen zur Pflege der Haut bekannt. Retinol, Vitamin A, wirkt beispielsweise gegen übermäßige Verhornung und Pigmentierung der Haut. Ein Mangel an Vitamin A äußert sich deshalb in trockener, rauher, verhornter Haut und einer Atrophie der Schweißdrüsen. Der Zusatz von Vitamin A zu Kosmetika ist insbesondere bei der Behandlung alternder Haut, bei der eine stärkere Verhornung vorliegt, indiziert. Vitamin A ist somit auch ein kosmetischer Wirkstoff zur prophylaktischen Aknebehandlung. Vitamin A ist allerdings auch empfindliche gegen Luft, Wärme und vor allem Lichteinflüssen.

Liponsäure oder Coenzym Q10 sind weitere bekannte Wirkstoffe, die nachgewiesenermaßen eine pflegende und wiederaufbauende Wirkung insbesondere alternder Haut zeigen. Diese Wirkstoffe werden vorwiegend als Antioxidantien als Schutzsubstanzen gegen den Verderb der sie enthaltenden Zubereitungen verwendet. Dennoch ist bekannt, dass auch in der menschlichen und tierischen Haut unerwünschte Oxidationsprozesse auftreten können. Solche Prozesse spielen eine wesentliche Rolle bei der Hautalterung.

Diese hautpflegenden Wirkstoffe haben, da sie als Antioxidantien wirken, den Nachteil, dass sie durch äußere Einflüsse, insbesondere Lichteinflüsse, mit der Zeit zerstört werden.
Die Wirksamkeit kosmetischer und/oder dermatologischer Mittel und die darin enthaltenen Wirkstoffe sollte aber während der gesamten Haltbarkeitsdauer des Mittel gewährleistet sein. Da einige der kosmetischen und dermatologischen Wirkstoffe durch Licht, insbesondere UV-Licht, einen Abbau erfahren können, ist es Aufgabe der vorliegenden Erfindung diese Stoffe vor dem lichtbedingten Abbau zu schützen.

Aus DE 19903729 sind oberflächenaktive Citronensäureester zur Stabilisierung von Retinoiden bekannt. Nachteilig ist deren auf die Wirkstoffgruppe der Retinoide begrenzte Stabilisierung.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen. Der schädigende Einfluss der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)), nicht in ausreichendem Masse gegeben ist.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls, kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, dass auch ionische Spezies bei UV-Exposition entstehen, welche dann ihrerseits oxidativ in die biochemischen Prozesse einzugreifen vermögen.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie des Triazins und auch des 2-Phenylbenzimidazols handelt. Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, dass UVA-Strahlung zu einer Schädigung der elastischen und kollagenen Fasern des Bindegewebes führt, was die Haut vorzeitig altern lässt, und dass sie als Ursache zahlreicher phototoxischer und photoallergischer Reaktionen zu sehen ist.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen.

Der Hauptnachteil aller im UV-Bereich absorbierenden Dibenzoylmethanderivate ist eine gewisse Instabilität gegenüber UV-Strahlung, so dass diese Komponenten unter UV-Einfluß zu inaktiven Produkten zersetzt werden und für die UV-Absorption nicht mehr zur Verfügung stehen. Zubereitungen des Standes der Technik mit einem Gehalt an diesen Substanzen enthalten daher zweckmäßigerweise auch bestimmte UV-Stabilisatoren, wie beispielsweise Ethylhexyl-2-cyano-3,3-diphenylacrylat (Octocrylen) oder 4-Methylbenzylidencampher.

Aus EP 685221 sind UV-Lichtschutzfilter bekannt, wie die Kombination von 2,4,6-tris[p-((2'-ethylhexyl)oxycarbonyl)anilino]-1,3,5-triazine und 2-ethylhexyl-α-cyano-β-β-diphenylacrylat. Diese bekannten UV-Lichtfiltersubstanzen dienen einzig und allein dem Schutz der Haut vor schädigendem UV-Licht. Der Zusatz bekannter Antioxidatien soll zudem die UV-Schutzzubereitungen vor oxidativem Zerfall schützen. Eine Schutzwirkung der bekannten Lichtschutzfilter für fotolabile Wirkstoffe wird nicht offenbart.

Aufgabe der vorliegenden Erfindung ist es, Stabilisierungsmittel zum Schutze kosmetischer Zubereitungen selbst bzw. zum Schutz der fotolabile Wirkstoffe vor schädlichen Oxidationsprozessen bereit zu stellen.

Im Rahmen umfangreicher Untersuchungen zur Stabilisierung lichtempfindlicher Wirkstoffe hat sich herausgestellt, dass die erfindungsgemäße Kombination aus Triazinderivat und einer Verbindung aus der Verbindungsklasse der β, β-Diphenylacrylatderivate die gestellten Aufgaben löst.

Es war überraschend und für den Fachmann nicht vorauszusehen, dass die synergistische Kombination umfassend
(I) mindestens eine Lichtfiltersubstanz aus der Verbindungsklasse der Triazinderivate und
(II) mindestens eine Lichtfiltersubstanz aus der Verbindungsklasse der β, β-Diphenylacrylatderivate
zum Schutz fotolabiler Wirkstoffe verwendet werden kann.
Die erfindungsgemäße Kombination gewährleistet eine einwandfreie Stabilität der fotolabilen Wirkstoffe entsprechend der Haltbarkeit der sie enthaltenden Kosmetika.

Besonders bevorzugt ist die Verwendung einer Zusammensetzung bei der das Triazinderivat 2,4-bis-{[(2-ethylhexyloxy)-2-hydroxyl]-phenyl}-6-(4-methoxyphenyl)-(1,3,5)-triazine, bekannt unter dem Namen Tinosorb S, oder bei der das β, β-Diphenylacrylatderivat 2-Ethylhexyl-2-cyano-3-diphenylacrylat, bekannt unter dem Namen Octocrylen, ist. Ganz besonders bevorzugt ist eine Zusammensetzung aus Tinosorb S und 2-Ethylhexyl-2-cyano-3-diphenylacrylat.

Ganz besonders effektiv hat sich die synergistische Kombination von Tinosorb S und Octocrylen für die Verminderung des lichtinduzierten Abbaus fotolabiler Wirkstoffe erwiesen. Neben der guten Stabilisierung und der damit zusammenhängenden vorteilhaften Haltbarkeit und Lagerfähigkeit der diese Wirkstoffe enthaltenden Kosmetika, sind überraschenderweise auch nur geringe Mengen der Triazin- bzw. der β, β-Diphenylacrylatderivate einzusetzen, um eine gewünschte Stabilität zu erreichen. So reichen Mengen von 0,001 bis 5 Gew.%, bezogen auf die Gesamtmasse einer kosmetischen Zubereitung, aus, um eine langfristige Stabilität der fotolabilen Wirkstoffe zu garantieren.

Die Verwendung der erfindungsgemäßen Kombination zum Schutz fotolabiler Wirkstoffe in kosmetischen und/oder dermatologischen Zubereitungen ist eine Innovation in der Pflegekosmetik.

Es war überraschend und für den Fachmann nicht vorauszusehen, dass kosmetische und dermatologische Zubereitung enthaltend
(I) mindestens eine Verbindung aus der Verbindungsklasse der Triazinderivate,
(II) mindestens eine Verbindung aus der Verbindungsklasse der β, β-Diphenylacrylatderivate und
(III) mindestens einen photolabilen Wirkstoff, welcher die Funktionalitäten -C-S-, -C=C- konjugiert, -C=C- isoliert, oder / und-C=N- aufweist,
den Stand der Technik bereichern.

Insbesondere erweisen sich kosmetische und/oder dermatologische Zubereitungen, bei denen das Triazinderivat 2,4-bis-{[(2-ethylhexyloxy)-2-hydroxyl]-phenyl}-6-(4-methoxyphenyl)-(1,3,5)-triazine und/oder das β, β-Diphenylacrylatderivat 2-Ethylhexyl-2-cyano-3-diphenylacrylat ist, als äußerst stabil gegen lichtbedingten Abbau der fotolabilen Wirkstoffe.

Die Zubereitung enthalten ein oder mehrere Triazinderivate in einem Anteil von 0,01 bis 10 Gew.-% , bevorzugt 0,25 bis 5 Gew.-%, insbesondere 0,5 bis 2 Gew.-% , bezogen auf das Gesamtgewicht der Zubereitung. Der Anteil der β, β-Diphenylacrylatderivate in der Zubereitung beträgt 0,01 bis 10 Gew.-% , insbesondere 0,5 bis 6 Gew.%, bezogen auf das Gesamtgewicht der Zubereitung.

Liegen der oder die lichtempfindlichen Wirkstoffe in einer erfindungsgemäßen Formulierung vor, so sind sie gegen die durch Licht-Strahlung induzierte Zersetzung in hervorragender Weise geschützt. Insbesondere erweist sich die erfindungsgemäße Kombination in der Stabilität als zuverlässig für Wirkstoffe, die im Wellenlängenbereich von 300 bis 350 nm fotolabil sind.

Eine erstaunliche Eigenschaft der erfindungsgemäße Zubereitungen ist, dass diese sehr gute Vehikel für kosmetische oder dermatologische Wirkstoffe in die Haut sind, wobei die Wirkstoffe Funktionalitäten -C-S-, -C=C- konjugiert, -C=C- isoliert, und/oder -C=N-aufweisen, wodurch eine gewisse Fotolabilität vorgegeben ist. Insbesondere Wirkstoffe ausgewählt aus der Gruppe α-Liponsäure und deren Derivate, Retinol und deren Derivate sowie ungesättigte Fettsäuren, insbesondere solche mit 6-34 Kohlenstoffatomen, sind bevorzugte Wirkstoffe in kosmetischen Zubereitungen und werden durch die erfindungsgemäße Kombination wirksam vor licht induziertem Abbau geschützt.
Neben Retinol ist auch ein Retinylester, ausgenommen Ester einer Fettsäure mit 1-26 Kohlenwasserstoffatomen, ein geeigneter Wirkstoff.
Weitere besonders bevorzugte lichtempfindliche Wirkstoffe sind Nicotinsäureamid (Vitamin B₃) oder dessen Derivat, insbesondere das Coenzym Nicotinamid-Adenin-Dinucleotid, ungesättigte Monoterpene oder Sesquiterpene, biologische Signalstoffe, insbesondere Kinetin oder Melatonin, und/oder ungesättigte Fettsäure und hier insbesondere Linolsäure, Linolensäure, Arachidonsäure und/oder Sorbinsäure.

Kosmetische oder dermatologische Zubereitungen enthalten daher vorteilhaft einen oder mehrere dieser fotolabilen Wirkstoffe, die zumeist als Antioxidantien die Haut vor oxidativer Beanspruchung schützen können. Es ist daher vorteilhaft, Antioxidantien als Wirkstoffklasse zu verwenden, etwa dann, wenn eine kosmetische oder dermatologische Anwendung im Vordergrunde steht, wie die Bekämpfung der oxidativen Beanspruchung der Haut. Es ist aber auch günstig, die erfindungsgemäßen Zubereitungen mit einem Gehalt an einem oder mehreren Antioxidantien zu versehen, wenn die Zubereitungen einem anderen Zwecke dienen sollen, z.B. als Desodorantien oder Sonnenschutzmittel.

Besonders vorteilhaft werden die Antioxidantien gewählt aus der Gruppe, bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Camosin, L-Carnosin und deren Derivate (z.B. Anserin), Carotinoide, Carotine (z.B. α-Carotin, β-Carotin, Lycopin) und deren Derivate, Liponsäure, α-Liponsäure und deren Derivate (z.B. Dihydroliponsäure), Aurothioglucose, Propylthiouracil und andere Thiole (z.B. Thioglycerin, Thiosorbitol, Thioglycolsäure, Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Pentat-, Hexa-, Heptahioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol deren Derivate, Vitamin C und Derivate (z.B. Ascorbylpalmitate, Mg - Ascorbylphosphate, Ascorbylacetate), Isoascorbinsäure und ihre Derivate, Tocopherole und Derivate (z.B. Vitamin E - acetat), Vitamin A und Derivate (Vitamin A - palmitat) sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate und/oder Mannose und deren Derivate.

Die Menge der eingesetzten Wirkstoffe in den Zubereitungen beträgt vorzugsweise 0,001 bis 10 Gew.-%, besonders bevorzugt 0,01 - 5 Gew.-%, insbesondere 0,05 - 3 Gew.%, bezogen auf das Gesamtgewicht der Zubereitung.

Besonders bevorzugt wird α-Liponsäure als Wirkstoff in kosmetischen und/oder dermatologischen Zubereitungen gewählt. Der Anteil an α-Liponsäure beträgt dann 0,001 bis 5 Gew.-%, bevorzugt 0,01 bis 3 Gew.-%, insbesondere 0,05 bis 1 Gew.-% bezogen auf das Gesamtgewicht der Zubereitung.

Sofern Vitamin E und/oder dessen Derivate das oder die fotolabilen Wirkstoffe darstellen, ist es vorteilhaft, deren jeweiligen Anteil aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw deren Derivate das oder die Wirkstoffe darstellen, ist vorteilhaft, deren jeweiligen Anteil aus dem Bereich von 0,001 - 5 Gew.%, bevorzugt 0,02 bis 5 Gew.%, insbesondere 0,03 bis 1 Gew.-% bezogen auf das Gesamtgewicht der Formulierung, zu wählen.

Erfindungsgemäß können Wirkstoffe auch sehr vorteilhaft gewählt werden aus der Gruppe der lipophilen Wirkstoffe, insbesondere aus folgender Gruppe:
Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin B₁, das Vitamin B₂, das Vitamin B₆, das Vitamin D₁, aber auch Bisabolol, ungesättigte Fettsäuren, namentlich die essentiellen Fettsäuren (oft auch Vitamin F genannt), insbesondere die γ-Linolensäure, Ölsäure, Eicosapentaensäure, Docosahexaensäure und deren Derivate, Chloramphenicol, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und Pseudoceramide und so weiter.

Vorteilhaft ist es auch, die Wirkstoffe aus der Gruppe der rückfettenden Substanzen zu wählen, beispielsweise Purcellinöl®, Eucerit® und Neocerit®.

Besonders vorteilhaft werden der oder die Wirkstoffe ferner gewählt aus der Gruppe der NO-Synthasehemmer, insbesondere wenn die erfindungsgemäßen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut dienen sollen. Bevorzugter NO-Synthasehemmer ist das Nitroarginin.

Weiter vorteilhaft werden der oder die Wirkstoffe, gewählt aus der Gruppe, welche Catechine und Gallensäureester von Catechinen und wäßrige bzw. organische Extrakte aus Pflanzen oder Pflanzenteilen umfaßt, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw. Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R,3R)-3,3',4',5,7-Flavanpentaol) ist ein vorteilhafter Wirkstoff im Sinne der vorliegenden Erfindung.

Vorteilhaft sind ferner pflanzliche Auszüge mit einem Gehalt an Catechinen, insbesondere Extrakte des grünen Tees, wie z. B. Extrakte aus Blättern der Pflanzen der Spezies Camellia spec., ganz besonders der Teesorten Camellia sinenis, C. assamica, C. taliensis bzw. C. irrawadiensis und Kreuzungen aus diesen mit beispielsweise Camellia japonica.

Bevorzugte Wirkstoffe sind ferner Polyphenole bzw. Catechine aus der Gruppe (-)-Catechin, (+)-Catechin, (-)-Catechingallat, (-)-Gallocatechingallat, (+)-Epicatechin, (-)-Epicatechin, (-)-Epicatechin Gallat, (-)-Epigallocatechin, (-)-Epigallocatechingallat.

Auch Flavon und seine Derivate (oft auch kollektiv "Flavone" genannt) sind vorteilhafte Wirkstoffe im Sinne der vorliegenden Erfindung. Sie sind durch folgende Grundstruktur gekennzeichnet (Substitutionspostitionen angegeben):

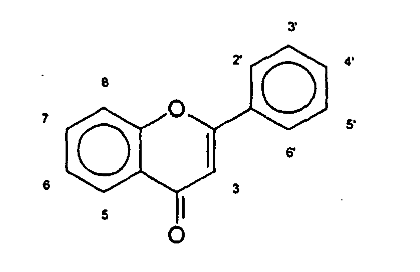

Einige der wichtigeren Flavone, welche auch bevorzugt in erfindungsgemäßen Zubereitungen eingesetzt werden können, sind in der nachstehenden Tabelle aufgeführt:

| | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kämpferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Erfindungsgemäß werden die Flavonoide bevorzugt gewählt aus der Gruppe der Substanzen der generischen Strukturformel 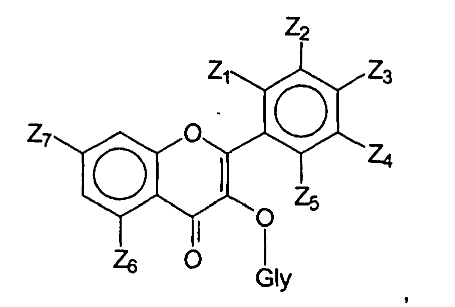 wobei Z₁ bis Z₇ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel 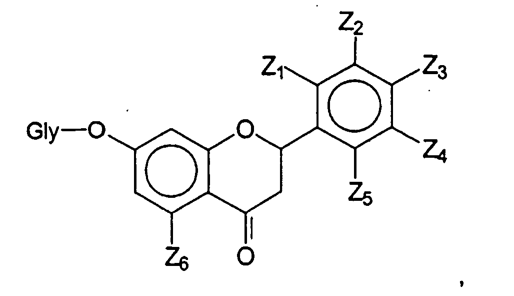 wobei Z₁ bis Z₆ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel 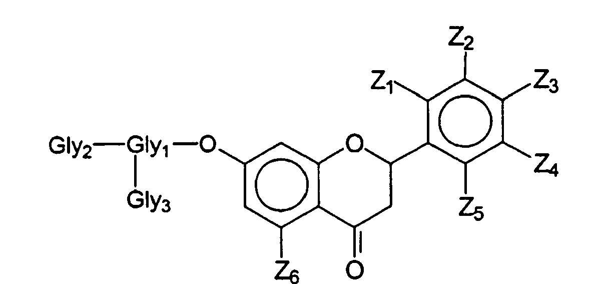 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden Z₁ bis Z₅ unabhängig voneinander gewählt aus der Gruppe H, OH, Methoxy-, Ethoxy- sowie 2-Hydroxyethoxy-, und die Flavonglycoside haben die Struktur

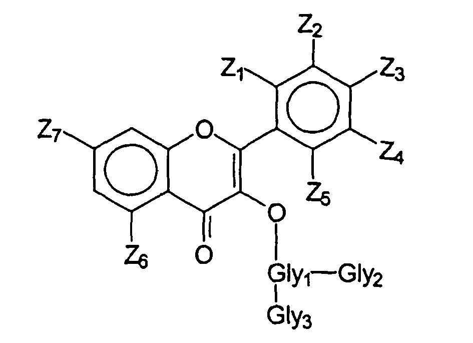

Besonders vorteilhaft werden die erfindungsgemäßen Flavonglycoside aus der Gruppe, welche durch die folgende Struktur wiedergegeben werden: 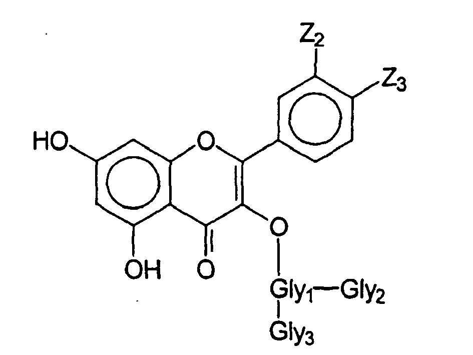 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin. Erfindungsgemäß besonders bevorzugt ist α-Glucosylrutin.

Erfindungsgemäß vorteilhaft sind auch Naringin (Aurantiin, Naringenin-7-rhamnoglucosid), Hesperidin (3',5,7-Trihydroxy-4'-methoxyflavanon-7-rutinosid, Hesperidosid, Hesperetin-7-O-rutinosid). Rutin (3,3',4',5,7-Pentahydroxyflyvon-3-rutinosid, Quercetin-3-rutinosid, Sophorin, Birutan, Rutabion, Taurutin, Phytomelin, Melin), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Dihydrorobinetin (3,3',4',5',7-Pentahydroxyflavanon), Taxifolin (3,3',4',5,7-Pentahydroxyflavanon), Eriodictyol-7-glucosid (3',4',5,7-Tetrahydroxyflavanon-7-glucosid), Flavanomarein (3',4',7,8-Tetrahydroxyflavanon-7-glucosid) und Isoquercetin (3,3',4',5,7-Pentahydroxyflavanon-3-(β-D-Glucopyranosid).
Auch Kreatin und/oder Kreatinderivate sind bevorzugte Wirkstoffe im Sinne der vorliegenden Erfindung. Kreatin zeichnet sich durch folgende Struktur aus:

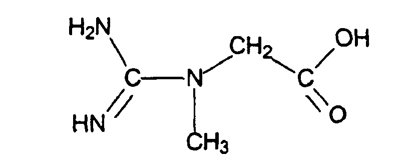

Bevorzugte Derivate sind Kreatinphosphat sowie Kreatinsulfat, Kreatinacetat, Kreatinascorbat und die an der Carboxylgruppe mit mono- oder polyfunktionalen Alkoholen veresterten Derivate.

Ein weiterer vorteilhafter Wirkstoff ist L-Carnitin [3-Hydroxy-4-(trimethylammonio)-buttersäurebetain]. Auch Acyl-Carnitine, welche gewählt aus der Gruppe der Substanzen der folgenden allgemeinen Strukturformel 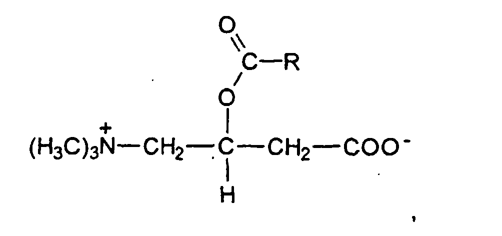 wobei R gewählt wird aus der Gruppe der verzweigten und unverzweigten Alkylreste mit bis zu 10 Kohlenstoffatomen sind vorteilhafte Wirkstoffe im Sinne der vorliegenden Erfindung. Bevorzugt sind Propionylcarnitin und insbesondere Acetylcarnitin. Beide Entantiomere (D- und L-Form) sind vorteilhaft im Sinne der vorliegenden Erfindung zu verwenden. Es kann auch von Vorteil sein, beliebige Enantiomerengemische, beispielsweise ein Racemat aus D- und L-Form, zu verwenden.
Weitere vorteilhafte Wirkstoffe sind Sericosid, Aminoguadin, Phytochelatin, Isoflavone (Genistein, Daidzein, Daidzin, Glycitin), Niacin, Tyrosinsulfat, Dioic Acid, Adenosin, Pyridoxin, Arginin, Vitamin K, Biotin und Aromastoffe, Sericosid wie auch Wirkstoffkombinationen der genannten Wirkstoffe.

Weitere vorteilhafte Wirkstoffe sind Phytomenadion oder 8-Hexadecen-1,16-dicarbonsäure.

Die Liste der genannten Wirkstoffe bzw. Wirkstoffkombinationen, die in den erfindungsgemäßen Zubereitungen verwendet werden können, soll selbstverständlich nicht limitierend sein. Die Wirkstoffe können einzelnen oder in beliebigen Kombinationen miteinander verwendet werden.

Die Zubereitungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W- oder O/W/O-Emulsionen vorliegen. Solche Formulierungen können vorzugsweise auch eine Mikroemulsion (z. B. eine PIT-Emulsion), eine Feststoff-Emulsionen (d. h. eine Emulsion, welche durch Feststoffe stabilisiert ist, z. B. eine Pickering-Emulsion), eine sprühbare Emulsion, ein Emulsionsstift, eine Hydrodispersion, eine emulgatorfreie Formulierung, eine wasserfreie Formulierung oder eine ölfreie Formulierung sein.

Erfindungsgemäss sind Emulsionen vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Die Lipidphase kann vorteilhaft gewählt werden aus folgender Substanzgruppe: Mineralöle,Mineralwachse, Öle, wie Triglyceride der Caprin- oder der Caprylsäure, ferner natürliche Öle wie z.B. Rizinusöl; Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren; Alkylbenzoate; Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C12-15-Alkylbenzoat, Capryl-Caprinsäure-triglycerid, Dicaprylylether.

Besonders vorteilhaft sind Mischungen aus C12-15-Alkylbenzoat und 2-Ethylhexylisostearat, Mischungen aus C12-15-Alkylbenzoat und Isotridecylisononanoat sowie Mischungen aus C12-15-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, ausser dem Silikonöl oder den Silikonölen einen zusätzlichen Gehalt an anderen Ölphasenkomponenten zu verwenden.
Vorteilhaft wird Cyclomethicon (Octamethylcyclotetrasiloxan) als erfindungsgemäss zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der Zubereitungen gemäss der Erfindung enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, beziehungsweise aus der Gruppe der sogenannten Pemulene, beispielsweise Pemulene der Typen TR-1, TR-2, jeweils einzeln oder in Kombination.

Insbesondere werden Gemische der vorstehend genannten Lösemittel verwendet. Bei alkoholischen Lösemitteln kann Wasser ein weiterer Bestandteil sein.

Emulsionen gemäss der Erfindung enthalten vorteilhaft z.B. die genannten Fette, Öle, Wachse und anderen Fettkörper, sowie Wasser und gegebenenfalls einen oder mehrere weitere Emulgatoren, wie sie üblicherweise verwendet werden.

Als Emulsionen vorliegende Zubereitungen gemäss der Erfindung enthalten gegebenenfalls besonders vorteilhaft einen oder mehrere zusätzliche O/W-Emulgatoren. Solche O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
der Fettalkoholethoxylate
der ethoxylierten Wollwachsalkohole,
der Polyethylenglycolether der allgemeinen Formel R-O-(-CH2-CH2O-)n-R,
der Fettsäureethoxylate der allgemeinen Formel R-COO-(-CH2-CH2-O-)n-H,
der veretherten Fettsäüreethoxylate der allgemeinen Formel R-COO-(-CH2-CH2-O-)n-R,
der veresterten Fettsäureethoxylate der allgemeinen Formel R-COO-(-CH2-CH2-O-)n-C(O)-R,
der Polyethylenglycolglycerinfettsäureester
der ethoxylierten Sorbitanester
der Cholesterinethoxylate
der ethoxylierten Triglyceride
der Alkylethercarbonsäuren der allgemeinen Formel R-O-(-CH2-CH2-O-)n-CH2-COOH wobei n eine Zahl von 5 bis 30 darstellen,
der Polyoxyethylensorbitolfettsäureester,
der Alkylethersulfate der allgemeinen Formel R-O-(-CH2-CH2-O-)n-SO3-H,
der Fettalkoholpropoxylate der allgemeinen Formel R-O-(-CH2-CH(CH3) -O-)n-H,
der Polypropylenglycolether der allgemeinen Formel R-O-(-CH2-CH(CH3)-O-)n-R,
der propoxylierten Wollwachsalkohole,
der veretherten Fettsäurepropoxylate R-COO-(-CH2-CH(CH3)-O-)n-R,
der veresterten Fettsäurepropoxylate der allgemeinen Formel R-COO-(CH2-CH(CH3)-O-)n-C(O)-R,
der Fettsäurepropoxylate der allgemeinen Formel R-COO-(-CH2-CH(CH3)-O-)n-H,
der Polypropylenglycolglycerinfettsäureester
der propoxylierten Sorbitanester
der Cholesterinpropoxylate
der propoxylierten Triglyceride
der Alkylethercarbonsäuren der allgemeinen Formel R-O-(-CH2-CH(CH3)O-)n-CH2-COOH,
der Alkylethersulfate bzw. die diesen Sulfaten zugrundeliegenden Säuren der allgemeinen Formel R-O-(-CH2-CH(CH3)-O-)n-SO3-H,
der Fettalkoholethoxylatelpropoxylate der allgemeinen Formel R-O-Xn-Ym-H,
der Polypropylenglycolether der allgemeinen Formel R-O-Xn-Ym-R,
der veretherten Fettsäurepropoxylate der allgemeinen Formel R-COO-Xn-Ym-R,
der Fettsäureethoxylate/propoxylate der allgemeinen Formel R-COO-Xn-Ym-H,.

Erfindungsgemäss besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit mit HLB-Werten von 14,5 - 15,5, sofern die O/W-Emulgatoren gesättigte Reste R und R aufweisen. Weisen die O/W-Emulgatoren ungesättigte Reste R und/oder R auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13), Polyethylenglycol(14)stearylether (Steareth-14), Polyethylenglycol(15)stearylether (Steareth-15), Polyethylenglycol(16),
stearylether (Steareth-16), Polyethylenglycol(17)stearylether (Steareth-17),
Polyethylenglycol(18)stearylether (Steareth-18), Polyethylenglycol(19)stearylether (Steareth-19),
Polyethylenglycol(20)stearylether (Steareth-20),
Polyethylenglycol(12)isostearylether (Isosteareth-12),
Polyethylenglycol(13)isostearylether (Isosteareth-13),
Polyethylenglycol(14)isostearylether (Isosteareth-14),
Polyethylenglycol(15)isostearylether (Isosteareth-15),
Polyethylenglycol(16)isostearylether (Isosteareth-16),
Polyethylenglycol(17)isostearylether (Isosteareth-17),
Polyethylenglycol(18)isostearylether (Isosteareth-18),
Polyethylenglycol(19)isostearylether (Isosteareth-19),
Polyethylenglycol(20)isostearylether (Isosteareth-20),
Polyethylenglycol(13)cetylether (Ceteth-13), Polyethylenglycol(14)cetylether (Ceteth-14),
Polyethylenglycol(15)cetylether (Ceteth-15), Polyethylenglycol(16)cetylether (Ceteth-16),
Polyethylenglycol(17)cetylether (Ceteth-17), Polyethylenglycol(18)cetylether (Ceteth-1,8),
Polyethylenglycol(19)cetylether (Ceteth-19), Polyethylenglycol(20)cetylether (Ceteth-20),
Polyethylenglycol(13)isocetylether (Isoceteth-13), Polyethylenglycol(14)isocetylether (Isoceteth-14), Polyethylenglycol(15)isocetylether (Isoceteth-15),
Polyethylenglycol(16)isocetylether (lsoceteth-16), Polyethylenglycol(17)isocetylether (Isoceteth-17), Polyethylenglycol(18)isocetylether (Isoceteth-18),
Polyethylenglycol(19)isocetylether (Isoceteth-19), Polyethylenglycol(20)isocetylether (Isoceteth-20),
Polyethylenglycol(12)oleylether (Oleth-12), Polyethylenglycol(13)oleylether (Oleth-13),
Polyethylenglycol(14)oleylether (Oleth-14), Polyethylenglycol(15)oleylether (Oleth-15),
Polyethylenglycol(12)laurylether (Laureth-12), Polyethylenglycol(12)isolaurylether (Isolaureth-12).
Polyethylenglycol(13)cetylstearylether (Ceteareth-13),
Polyethylenglycol(14)cetylstearylether (Ceteareth-14),
Polyethylenglycol(15)cetylstearylether (Ceteareth-15),
Polyethylenglycol(16)cetylstearyfether (Ceteareth-16),
Polyethylenglycol(1 7)cetylstearylether (Ceteareth-17),
Polyethylenglycol(18)cetylstearylether (Ceteareth-18),
Polyethylenglycol(19)cetylstearylether (Ceteareth-19),
Polyethylenglycol(20)cetylstearylether (Ceteareth-20),

Es ist ferner von Vorteil, die Fettsäureethoxylate aus folgender Gruppe zu wählen:
Polyethylenglycol(20)stearat, Polyethylenglycol(21)stearat, Polyethylenglycol(22)stearat,
Polyethylenglycol(23)stearat, Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat, Polyethylenglycol(14)oleat,
Polyethylenglycol(15)oleat, Polyethylenglycol(16)oleat, Polyethylenglycol(17)oleat,
Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natrium-laureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als Emulsionen vorliegende Zubereitungen gemäss der Erfindung enthalten aber auch gegebenenfalls vorteilhaft einen oder mehrere zusätzliche W/O-Emulgatoren. Als solche vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Gele gemäss der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wässrig-alkoholischen oder alkoholischen Gelen vorzugweise ein Polyacrylat ist.

Erfindungsgemäss können die kosmetischen und dermatologischen Zubereitungen kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Es ist auch möglich und vorteilhaft im Sinne der vorliegenden Erfindung, erfindungsgemäss verwendete Zubereitung in wässrige Systeme bzw. Tensidzubereitungen zur Reinigung der Haut und der Haare einzufügen.

Zur Anwendung werden die kosmetischen und dermatologischen Zubereitungen erfindungsgemäss in der für Kosmetika üblichen Weise auf die Haut und/oder die Haare in ausreichender Menge aufgebracht.

### Beispiele 1-10: O/W-Cremes

| **Beispiel Nummer** | **1** | **2** | **3** | **4** | **5** |
|---|---|---|---|---|---|
| Glycerylstearatcitrat | 2 | | | 2 | |
| Glycerylstearat, selbstemulgierend | | 5 | 3 | | 2 |
| PEG-40-Stearat | | | 1 | | 1 |
| Myristylmyristat | 1 | | | | 1 |
| Behenylalkohol | | | | | |
| Stearylalkohol | 2 | 1 | | | |
| Cetearylalkohol | | | | 4 | 2 |
| Cetylalkohol | 1 | | 3 | | |
| Hydrierte Kokosfettglyceride (Hydrogenated Coco Glycerides) | 2 | | | | |
| Sheabutter | | 2 | | | 2 |
| C12-15 Alkylbenzoat | | 3 | 2 | | 3 |
| Butylenglycol Dicaprylat/Dicaprat | 1 | | | 1 | |
| Caprylsäure/Caprinsäure Triglycerid | | 1 | 1 | 2 | 2 |
| Ethylhexylkokosfettsäureester | 3 | | | | 1 |
| Octyldodecanol | | | 1 | | |
| Mineralöl | | 1 | | | |
| Vaseline | 2 | | 1 | | 2 |
| Octamethyltetrasiloxan (Cyclomethicon) | 4 | 1 | 4 | 3 | 5 |
| Dimethylpolysiloxan (Dimethicon) | | | 1 | 1 | |
| Dicaprylylether | 1 | 4 | 2 | | |
| Dicarprylylcarbonat | | | | 3 | |
| TiO₂ | | | | 0,5 | |
| Ethylhexylmethoxycinnamat | | | | | 2 |
| Ethylhexylcyanodiphenylacrylat (Octocrylen) | 5 | 3 | 5 | 7 | 4 |
| Bis-Ethylhexyloxyphenolmethoxyphenyltriazine | 1 | 0,5 | 1 | 2 | 0,8 |
| Ethylhexylsalicylat | | | | | 1 |
| Kinetin | 0,1 | | | | |
| Biotin | | | | | 0,04 |
| Retinol | | | | 0,1 | |
| α-Liponsäure | | 0,3 | 0,2 | 0,5 | |
| Tocopherylacetat | | | 1 | | |
| Citronensäure, Natriumsalz | | 0.1 | | | |
| Natriumascorbylphosphat | 0,1 | | | | |
| Trinatrium EDTA | | 0.1 | | 0,2 | |
| lminodisuccinat, Natriumsalz | 0,2 | | 0,1 | | 0,1 |
| Phenoxyethanol | 0,3 | | 0,3 | 0,2 | 0,2 |
| p-Hydroxybenzoesäurealkylester (Paraben) | 0,6 | 0,3 | 0,2 | 0,3 | 0,3 |
| Hexamidindüsethionat | | 0,04 | | | |
| Diazolidinylharnstoff | 0,25 | | 0,1 | 0,2 | 0,1 |
| 1,3-Dimethylol-5,5-dimethylhydantoin | | 0,2 | | | |
| (DMDM Hydantoin) | | | | | |
| Iodopropynylbutylcarbamat | | 0,1 | | | |
| Ethanol denaturiert | | 2 | | | |
| Xanthan Gummi | 0,1 | | | | |
| Polyacrylsäure (Carbomer) | 0,05 | | 0,1 | | 0,1 |
| Polyacrylamid | | 0,2 | | | |
| Glycerin | 10 | 6 | 6 | 7,5 | 8 |
| Butylenglycol | 2 | 1 | | | |
| wasser- und/oder öllösliche Farbstoffe | 0,05 | | | | |
| Füllstoffe/ Additive (Distärkephosphat, SiO₂, BHT, Talkum, Aluminiumstearat) | 0,1 | 1 | 0,2 | 0,5 | 0,05 |
| Parfüm | q.s. | q.s. | q.s. | q.s. | q.s. |
| Wasser | Ad 100 | Ad 100 | Ad 100 | Ad 100 | Ad 100 |

| **Beispiel Nummer** | **6** | **7** | **8** | **9** | **10** |
|---|---|---|---|---|---|
| Glycerylsterat, selbstemulgierend | 2,5 | | | | |
| PEG-40-Stearat | 1 | | | | |
| Polyglyceryl-3-Methylglucosedistearat | | 3 | | | |
| Sorbitanstearat | | 1 | | | |
| Polyethylenglycol(21)stearylether (Steareth-21) | | | 2 | | |
| Polyethylenglycol(2)stearylether (Steareth-2) | | | 1 | | |
| Cetearylglucosid | | | | 2 | |
| Stearinsäure | | | | | 2,5 |
| Myristylmyristat | | | | 1 | |
| Behenylalkohol | | 1 | | | 2 |
| Stearylalkohol | | | | 5 | |
| Cetearylalkohol | 3 | | 2 | | 1 |
| Cetylalkohol | | 1 | | | |
| Hydrierte Kokosfettglyceride (Hydrogenated Coco Glycerides) | 1 | | | | 1 |
| Sheabutter | 2 | | | | |
| C12-15 Alkylbenzoat | 4 | 2 | 5 | 2 | |
| Butylenglycol Dicaprylat/Dicaprat | | | | | 2 |
| Caprylsäure/Caprinsäure Triglycerid | 1 | 1 | | 3 | |
| Hydriertes Polydecen | | | | 1 | |
| Ethylhexylkokosfettsäureester | | | | | 2 |
| Octyldodecanol | | | 1 | | 1 |
| Mineralöl | | | 1 | | |
| Vaseline | 1 | | | | |

| Octamethyltetrasiloxan (Cyclomethicon) | 4 | 3 | 2 | | 2 |
|---|---|---|---|---|---|
| Dimethylpolysiloxan (Dimethicon) | | | | | 1 |
| Dicaprylylether | | | 2 | | |
| Dicarprylylcarbonat | | 2 | | 3 4 | |
| Polydecen | | | | 4 | |
| TiO₂ | | | 1 | | |
| Ethylhexylmethoxycinnamat | | 3 | | | |
| Ethylhexylcyanodiphenylacrylat (Octocrylen) | 5 | 4 | 3 | 7 | 6 |
| Phenylbenzimidazolsulfonsäure | 2 | | | | 1 |
| Bis-Ethylhexyloxyphenolmethoxyphenyltriazine | 1 | 1 | 0,8 | 2 | 0,5 |
| Linolsäure | | | 0,2 | | |
| Tocopherylacetat | | 1 | | | 0,5 |
| Retinol | | | | 0,2 | |
| α-Liponsäure | 0,2 | 0,3 | | | 0,4 |
| Ascorbinsäure | | | 0,05 | | |
| Lactoferrin | | | | | 0,05 |
| Trinatrium EDTA | | | 0,2 | 0,1 | |
| Iminodisuccinat | 0,2 | 0,2 | | | 0,1 |
| Phenoxyethanol | 0,5 | 0,4 | 0,5 | | 0,3 |
| p-Hydroxybenzoesäurealkylester (Paraben) | 0,1 | | | 0,4 | 0,6 |
| Hexamidindüsethionat | | | 0,1 | | |
| Diazolidinylharnstoff | 0,2 | 0,2 | | 0,1 | |
| lodopropynylbutylcarbamat | | | 0,25 | | |
| Ethanol denaturiert | | 8 | | | 3 |
| 2-Ethylhexylglycerinether (Octoxyglycerin) | | | | 0,4 | |
| Xanthan Gummi | | 0,1 | | | |
| Polyacrylsäure (Carbomer) | 0,2 | | 0,1 | | 0,1 |
| Polyacrylamid | | 0,2 | | | |
| Glycerin | 10 | 5 | 6 | 4 | 7 |
| Butylenglycol | | | | 2 | |
| Wasser- und/oder öllösliche Farbstoffe | | | | | 0,1 |
| Additive (Distärkephosphat, SiO₂, Talkum, BHT Aluminiumstearat) | 0.03 | 0,1 | 0,05 | 3 | 1 |
| Parfüm | q.s. | q.s. | q.s. | q.s. | q.s. |
| Wasser | Ad 100 | Ad 100 | Ad 100 | Ad 100 | Ad 100 |

### Beispiel 11: W/O-Creme

| | |
|---|---|
| Polyglyceryl-3-Diisostearat | 5,0 |
| Polyglyceryl-2-Dipolyhydroxystearat | 2,5 |
| Cetearylalkohol | 2 |
| Cetylalkohol | 2 |
| C12-15 Alkylbenzoat | 8 |
| Caprylsäure/Caprinsäure Triglycerid | 6 |
| Octyldodecanol | 5 |
| Octamethyltetrasiloxan (Cyclomethicon) | 2 |
| Milchsäure | 5 |
| Citronensäure, Natriumsalz | 0,5 |
| Ethylhexylcyanodiphenyl-acrylat (Octocrylen) | 5 |
| Bis-Ethylhexyloxyphenol-methoxyphenyltriazin | 1 |
| α-Liponsäure | 0,3 |
| Phenoxyethanol | 0,1 |
| p-Hydroxybenzoesäurealkyleste (Paraben) | 0,1 |
| Glycerin | 7.5 |
| Füllstoffe (Distärkephosphat, SiO₂, Talkum, Aluminiumstearat) | 0,2 |
| Parfüm | q.s. |
| Wasser | Ad 100 |

### Beispiel 12: Hydrodispersion/ Gelcreme

| | |
|---|---|
| Cetylalkohol | 2 |
| Sheabutter | 1 |
| Caprylsäure/Caprinsäure Triglycerid | 2 |
| Octyldodecanol | 1 |
| Octamethyltetrasiloxan (Cyclomethicon) | 5 |
| Dimethylpolysiloxan (Dimethicon) | 1 |
| Polydecen | 2 |
| Ethylhexylmethoxycinnamat | 3 |
| Bis-Ethylhexyloxyphenol-methoxyphenyltriazin | 0,5 |
| Ethylhexylcyanodiphenyl-acrylat (Octocrylen) | 4 |
| α-Liponsäure | 0,1 |
| Iminodisuccinat | 0,2 |
| Phenoxyethanol | 0,3 |
| p-Hydroxybenzoesäurealkyleste (Paraben) | 0,4 |
| Vernetztes Alkylacrylat (Alkylacrylate Cosspolymer) | 0,2 |
| Glycerin | 5 |
| Parfüm | q.s. |
| Wasser | Ad 100 |

### Beispielrezeptur Foundations

| | | | | |
|---|---|---|---|---|
| **Inhaltsstoffe** | **1** | **2** | **3** | **4** |
| PEG-30-stearat | 1,5 | | | |
| Gylcerylstearat | 0,5 | | | 2 |
| Ceteareth-20 | 1 | | | |
| Polyglyceryl-3 Methylglucose stearat | | | 3,5 | |
| Sorbinsäurestearat | | | 1,5 | |
| Stearinsäure | 2 | | | 1,8 |
| Glycerylstearatcitrat | | 3,5 | | |
| Cetylalkohol | 0,5 | 0,75 | 1,0 | |
| Lecithin | | | | 0,5 |
| Veegum K = Mg-Al-Silicate | 0,8 | | | 1 |
| Xanthan Gum | 0,2 | | | 0,3 |
| Carbomer | | 0,2 | | |
| Hydroxyethylcellulose | 0,2 | | 0,1 | |
| Caprylsäure / Caprinsäure Triglycerid | | 2 | 3 | 2 |
| Dicaprylylether | | 2 | 3 | 3 |
| Octyldodecanol | | | 3 | |
| Dimethicon | 3 | | 3 | 3 |
| Cyclomethicon | | 4 | 3 | 2 |
| C12-C15 Alkyl Benzoate | 2 | | | |
| Cetearyloctanoat | 2 | | | |
| Squalan | 1 | | | 6 |
| Isopropylpalmitat | 1 | | | 2 |
| PPG -15 Stearylether | 2 | 3 | | |
| Hydrierete Kokos-Glyceride | 2 | | | |
| Stearyldimethicon | 9 | | | |
| Acetyliertes Lanolinöl | 2 | 0,2 | | |
| Ethylhexylmethoxycinnamat | | | 2 | |
| Ethylhexylcyanodiphenyl-acrylat (Octocrylen) | 2 | 5 | 6 | 5 |
| Bis-Ethylhexyloxyphenolmethoxyphenyltriazin | 0,5 | 1 | 2 | 0,5 |
| Ubichinon (Q10) | | | 0,02 | |
| Niacinamid | 0,1 | | | |
| α-Liponsäure | 0,2 | 0,2 | | 0.5 |
| Silikon | | 0,8 | | |
| Nylon-12 | | | 5 | |
| Lauroyllysin | 0,5 | | 0,5 | |
| Kaolin | 0,5 | | 1 | 2 |
| Natrium Stärke Octenylsuccinat | | 1 | | 1,5 |
| Eisenoxide | 1,2 | 2,4 | 2,6 | 3 |
| Titandioxid | 3,8 | 5,6 | 4,5 | 6,5 |
| Interferenz Pigmente | 0,2 | | | 0,5 |
| Pigment Low Lustre | 0,2 | | | 0,2 |
| ZnO | | 1 | | |
| Polymethylsilsesquioxan (Tospearl 2000B) | | 2 | | |
| EDTA | 0,1 | 0,6 | 1 | 1 |
| Glycerin | 2 | 5 | 5 | 10 |
| Phenoxyethanol und Paraben (Phenonip) | 1 | 0,5 | | 1 |
| Imidazonidinylharnstoff | 0,3 | 0,3 | | 0,25 |
| Neutralisationsmittel | q.s. | q.s. | q.s. | q.s. |
| Parfum, Antioxidantien | q.s. | q.s. | q.s. | q.s. |
| Wasser | ad 100 | ad 100 | ad 100 | ad 100 |

Alle beispielhaft aufgeführten Zubereitungen erweisen sich als sehr stabil gegenüber Lichteinflüssen und insbesondere längerer UV-Bestrahlung ohne relevante Einbußen hinsichtlich Wirksamkeit, Pflege, Anwendungseigenschaften oder Sensorik. Der lichtbedingte Abbau der Wirkstoffe mit den Strukturparametern -C-S-, -C=C- konjugiert, - C=C- isoliert, oder / und-C=N- wird reduziert und damit die Bildung unerwünschter Abbauprodukte deutlich reduziert.

## Patentansprüche

1. Verwendung einer Zusammensetzung umfassend
(I) mindestens eine Lichtfiltersubstanz aus der Verbindungsklasse der Triazinderivate und
(II) mindestens eine Lichtfiltersubstanz aus der Verbindungsklasse der β, β-Diphenylacrylatderivate
zum Schutz fotolabiler Wirkstoffe, **dadurch gekennzeichnet, dass** die Wirkstoffe Funktionalitäten -C-S-, -C=C- konjugiert, -C=C- isoliert, oder/und-C=N- aufweisen und gewählt werden aus der Gruppe der α-Liponsäure oder deren Derivate, Retinol oder deren Derivate und/oder einer mehrfach ungesättigten Fettsäure, insbesondere ungesättigte Fettsäuren mit 6-34 Kohlenstoffatomen.

2. Kosmetische oder dermatologische Zubereitungen umfassend
(I) mindestens eine Verbindung aus der Verbindungsklasse der Triazinderivate,
(II) mindestens eine Verbindung aus der Verbindungsklasse der β, β-Diphenylacrylatderivate und
(III) mindestens einen photolabilen Wirkstoff,
**dadurch gekennzeichnet, dass** die Wirkstoffe Funktionalitäten -C-S-, -C=C-konjugiert, -C=C isoliert, oder/und-C=N- aufweisen und gewählt werden aus der Gruppe der α-Liponsäure oder deren Derivate, Retinol oder deren Derivate und/oder einer mehrfach ungesättigten Fettsäure, insbesondere ungesättigte Fettsäuren mit 6-34 Kohlenstoffatomen.

## Claims

1. Use of a composition comprising
(I) at least one photofilter substance from the compound class of triazine derivatives and
(II) at least one photofiltez substance from the compound class of β,β-diphenylacrylate derivatives
for protecting photolabile active ingredients, **characterized in that** the active ingredients have functionalities -C-S-, -C=C- conjugated, -C=C- isolated, and/or -C=N- and are selected from the group of α-lipoic acid or derivatives thereof, retinol or derivatives thereof and/or a polyunsaturated fatty acid, in particular unsaturated fatty acids with 6-34 carbon atoms.

2. Cosmetic or dermatological preparations comprising
(I) at least one compound from the compound class of triazine derivatives,
(II) at least one compound from the compound class of β,β-diphenylacrylate derivatives and
(III) at least one photolabile active ingredient,
**characterized in that** the active ingredients have functionalities -C-S-, -C=C-conjugated, -C=C- isolated, and/or -C=N-and are selected from the group of α-lipoic acid or derivatives thereof, retinol or derivatives thereof and/or a polyunsaturated fatty acid, in particular unsaturated fatty acids with 6-34 carbon atoms.

## Revendications

1. Utilisation d'une composition contenant
(I) au moins une substance filtrant la lumière, choisie dans la classe de composés des dérivés de triazine et
(II) au moins une substance filtrant la lumière, choisie dans la classe de composés des dérivés de β-β-diphénylacrylate
pour la protection de substances actives photolabiles, **caractérisée en ce que** les substances actives présentent des fonctionnalités -C-S-, -C=C- conjuguées, -C=C- isolées et/ou -C=Net sont choisies dans le groupe constitué par l'acide α-lipoique ou ses dérivés, le rétinol ou ses dérivés et/ou un acide gras plusieurs fois insaturé, en particulier des acides gras insaturés ayant de 6 à 34 atomes de carbone.

2. Préparations cosmétiques ou dermatologiques, comprenant
(I) au moins un composé choisi dans la classe de composés des dérivés de triazine,
(II) au moins un composé choisi dans la classe de composés des dérivés de β,β-diphénylacrylate et
(III) au moins une substance active photolabile
**caractérisées en ce que** les substances actives présentent des fonctionnalités -C-S-, -C=C-conjuguées, -C=C- isolées et/ou -C=N- et sont choisies dans le groupe constitué par l'acide α-lipoïque ou ses dérivés, le rétinol ou ses dérivés et/ou un acide gras plusieurs fois insaturés, en particulier des acides gras insaturés ayant de 6 à 34 atomes de carbone.
